Europäisches Patentamt

European Patent Office

Office européen des brevets

(11) Veröffentlichungsnummer: **0 251 195 B1**

## EUROPÄISCHE PATENTSCHRIFT

(45) Veröffentlichungstag der Patentschrift: **03.02.93**

(21) Anmeldenummer: **87109113.8**

(22) Anmeldetag: **24.06.87**

(51) Int. Cl.5: **C12Q 1/40**, C12P 21/00, C12N 5/00, C12N 15/00, G01N 33/573

(54) Verfahren und Reagenz zur Bestimmung von alpha-Amylase.

(30) Priorität: **25.06.86 DE 3621271**

(43) Veröffentlichungstag der Anmeldung:
**07.01.88 Patentblatt 88/01**

(45) Bekanntmachung des Hinweises auf die
Patenterteilung:
**03.02.93 Patentblatt 93/05**

(84) Benannte Vertragsstaaten:
**AT BE CH DE ES FR GB GR IT LI LU NL SE**

(56) Entgegenhaltungen:
**EP-A- 0 135 758      EP-A- 0 171 960
EP-A- 0 191 284      EP-A- 0 194 600
EP-A- 0 209 154      US-A- 4 304 854**

(73) Patentinhaber: **BOEHRINGER MANNHEIM
GMBH
Patentabteilung, Abt. E Sandhofer Strasse
112-132 Postfach 31 01 20
W-6800 Mannheim 31 Waldhof(DE)**

(72) Erfinder: **Gerber, Martin, Dr. rer. nat.
Ignaz-Manz-Strasse 1
W - 8120 Weilheim-Unterhausen(DE)**

(74) Vertreter: **Huber, Bernhard, Dipl.-Chem. et al
Patentanwälte H. Weickmann, Dr. K. Fincke
F.A. Weickmann, B. Huber Dr. H. Liska, Dr. J.
Prechtel Kopernikusstrasse 9 Postfach 86 08
20
W-8000 München 86(DE)**

**Beschreibung**

Die Erfindung betrifft ein Verfahren und ein Reagenz zur Bestimmung der alpha-Amylase durch Spaltung eines Substrats derselben in Gegenwart eines oder mehrerer Hilfsenzyme und Messung eines Spaltproduktes.

Die Bestimmung der alpha-Amylase sowie ihrer Isoenzyme gehört zu den besonders wichtigen Methoden der klinischen Chemie, da ihre Ergebnisse zu diagnostischen Zwecken, insbesondere zur Diagnose von Pankreaserkrankungen verwendet werden. Infolgedessen wurde eine Vielzahl von Verfahren zur Bestimmung der alpha-Amylase entwickelt. Sie beruhen zum großen Teil auf der Spaltung eines Substrats, häufig in Gegenwart eines oder mehrerer Hilfsenzyme, und Messung eines Spaltproduktes. Technische Bedeutung haben u.a. mit bestimmbaren Gruppen derivatisierte Stärke sowie Maltooligoside erlangt, wie sie z.B. in den US-PSen 3,879,263 und 4,000,042 und in der DE-OS 27 41 192 beschrieben werden. Die erwähnten Maltooligoside können auch durch optisch bestimmbare Gruppen derivatisiert sein, wie beispielsweise in den DE-OSen 27 31 421 und 27 55 803 beschrieben wird, oder Ethylidenschutzgruppen aufweisen (EP 135758 A1).

Eine generelle Schwierigkeit all dieser Bestimmungen liegt darin, daß die Substrate verschiedene Angriffspunkte für die Spaltung durch die alpha-Amylase aufweisen und daher unterschiedliche Spaltprodukte entstehen, teilweise nach recht komplizierten Mechanismen. Hierdurch wird die exakte Berechnung der Aktivitäten erheblich erschwert und teilweise in der Genauigkeit beeinträchtigt. Beispielsweise entsteht bei der Spaltung des Substrats alpha-(4-Nitrophenyl)-Maltoheptaose etwa 60 % Maltotetraose, welches durch die nachfolgende Indikatorreaktion, die beispielsweise mit alpha-Glucosidase durchgeführt wird, nur schlecht gespalten wird. Analoge Schwierigkeiten liegen bei den anderen Maltooligosid-Substraten und ihren Derivaten vor. Die Schwierigkeiten würden sich beseitigen lassen, wenn es gelänge, die Spaltung der Substrate so ablaufen zu lassen, daß ausschließlich Maltotriose und Maltose bzw. deren entsprechende Derivate entstehen würden.

Darüberhinaus wäre es erwünscht, wenn man auch die Spaltgeschwindigkeit der Substrate erhöhen könnte.

Der Erfindung liegt daher die Aufgabe zugrunde, die oben bezeichneten Schwierigkeiten bei den bisher angewendeten Substraten zu beseitigen und insbesondere ein verbessertes Spaltmuster der Substrate sowie eine erhöhte Spaltrate zu erzielen.

Gelöst wird diese Aufgabe erfindungsgemäß durch ein Verfahren zur Bestimmung von alpha-Amylase durch Spaltung eines Substrats in Gegenwart eines oder mehrerer Hilfsenzyme und Messung eines Spaltproduktes, welches dadurch gekennzeichnet ist, daß man die Umsetzung in Gegenwart von monoklonalen Antikörpern gegen alpha-Amylase durchführt und hierbei mindestens einen monoklonalen Antikörper verwendet, welcher in der Lage ist, alpha-Amylase in Gegenwart der monoklonalen Antikörper zu binden, die von den Hybridzellklonen NCACC 84111301, NCACC 84122003, NCACC 85022203 und ECACC 86060601 gebildet werden.

Aus der älteren nicht vorveröffentlichten europäischen Anmeldung EP-A 0 191 284 ist ein Verfahren zur spezifischen Bestimmung von Pankreas-$\alpha$-Amylase neben Speichel-$\alpha$-Amylase bekannt, bei dem ein monoklonaler Antikörper verwendet wird, welcher das Speichel-Enzym spezifisch hemmt, das Pankreas-Enzym aber gar nicht oder nur bis 50 % inhibiert. Ebenso ist aus der älteren nicht vorveröffentlichten europäischen Anmeldung EP-A 0 209 154 ein Verfahren derselben Art bekannt, bei welchem man eine Kombination von zwei monoklonalen Antikörpern verwendet, von denen einer das Speichelenzym zu weniger als 97 % spezifisch hemmt und der andere dieses Enzym zu weniger als 10 % hemmt. Demgegenüber werden gemäß vorliegender Erfindung monoklonale Antikörper verwendet, welche nicht zwischen den beiden Isoenzymen der $\alpha$-Amylase diskriminieren, sondern an ein anderes Epitop als die bekannten monoklonalen Antikörper binden und hierbei das Enzym nicht hemmen, sondern im Gegenteil aktivieren.

Als Substrate können für das erfindungsgemäße Verfahren die natürlichen Substrate der alpha-Amylase und deren Derivate verwendet werden, beispielsweise Stärkederivate oder Oligo- und Polysaccharide. Als besonders geeignet erwiesen sich die D-Maltooligoside mit 2 bis 10 Glucoseeinheiten im Molekül, welche gegebenenfalls durch eine chromophore Gruppe substituiert sein können oder/und mindestens eine Ethylidenschutzgruppe tragen können. Besonders bevorzugt werden solche D-Maltooligoside, die 4 bis 8 Glucoseeinheiten im Molekül enthalten. Geeignete chromophore Gruppen, die nach Abspaltung leicht optisch bestimmt werden können, sind beispielsweise die Phenyl-, Mononitrophenyl-, Sorbit- oder Gluconsäuregruppe (DE-OS 27 55 803). Ebenso kommen Farbstoffe oder Farbstoffkupplungskomponenten als Substituenten in Betracht, beispielsweise Fluoreszenzfarbstoffe oder Vorläufer derselben, die sich leicht zum eigentlichen Farbstoff in bekannter Weise umwandeln lassen. Die Messung dieser Schutzgruppen ist dem Fachmann bekannt und bedarf hier keiner näheren Erläuterung. Typische Beispiele für im Rahmen des erfindungsgemäßen Verfahrens besonders geeignete Substrate sind Maltotetraose, Maltopentaose, Maltohexaose, Maltoheptaose und Maltooctaose, deren Mononitrophenyl- und die Dinitrophenylderivate, die Ethylidenderivate der aufgeführten Maltooligoosen und die entsprechenden Verbindungen, welche sowohl eine Ethylidenschutzgruppe als auch eine Nitrophenyl- oder Dinitrophenylgruppe aufweisen.

Die in Betracht kommenden Hilfsenzyme hängen von der Art des verwendeten Substrats ab und dienen dazu, einerseits durch die alpha-Amylase gebildete Spaltprodukte evtl. weiterzuspalten oder unter Bildung bestimmbarer Endprodukte weiterumzusetzen. So wird bei Verwendung der bevorzugten mit Nitrophenylgruppen substituierten Maltooligoside mit 2 bis 10 Glucoseresten als Hilfsenzym alpha-Glucosidase allein, wenn es sich um die alpha-Verbindungen handelt, oder zusammen mit $\beta$-Glucosidase, wenn es sich um die $\beta$-Verbindungen, wie z.B. $\beta$-(p-Nitrophenyl-)Maltoheptasaccharid handelt, verwendet. Im Falle der Maltoheptaose erfolgt die Spaltung durch alpha-Amylase unter Bildung von Maltotriose und Maltotetraose, die ihrerseits durch die alpha-Glucosidase völlig zu den Glucoseeinheiten gespalten wird. Die erhaltene Glucose kann dann nach einem der üblichen Glucosebestimmungsverfahren, beispielsweise mit Hexokinase und Glucose-6-Phosphatdehydrogenase als Hilfsenzymen gemessen werden.

Ebenso ist es möglich, das Maltooligosid, beispielsweise Maltoheptaose oder Maltotetraose, durch die alpha-Amylase bis zur Maltose spalten zu lassen und letztere mit Maltosephosphorylase, $\beta$-Phosphogluco-mutase und Glucose-6-Phosphatdehydrogenase in ebenfalls bekannter Weise zu bestimmen. Die genannten Enzyme werden dann als Hilfsenzyme beim Verfahren der Erfindung zugesetzt. Diese und weitere Bestimmungsmethoden für die Spaltprodukte der alpha-Amylase sind dem Fachmann bekannt und bedürfen hier daher keiner weiteren Erläuterung.

Als monoklonale Antikörper werden im Rahmen der Erfindung bevorzugt diejenigen verwendet, die von den Hybridzellenklonen 469A12 = ECACC 86020601 oder 472G12 = ECACC 86020602 gebildet werden. Die Erfindung kann jedoch auch mit anderen monoklonalen Antikörpern durchgeführt werden, welche gegen die alpha-Amylase gerichtet sind und in Gegenwart des oben angegebenen monoklonalen Antikörper binden. Diese monoklonalen Antikörper können durch die hierfür üblichen Verfahren erhalten werden, also insbesondere durch Verwendung von alpha-Amylase als Immunogen bei einem geeigneten Versuchsorganismus, Gewinnung und Fusionierung der gegen das Immunogen gerichtete Antikörper produzierenden Leukozyten mit Myelomzellen oder anderen die permanente Kultivierbarkeit bewirkenden Zellen oder Zellfragmenten und Züchtung und Auswahl der so erhaltenen Zellklone nach den gebildeten Antikörpern.

Als Versuchstiere für die Immunisierung können beispielsweise Balb/c-Mäuse verwendet werden, und die Immunisierung selbst kann mit kompletten oder nicht kompletten Freund'schen Adjuvants oder nur in physiologischer Kochsalzlösung erfolgen. Nach erfolgter Immunisierung werden die Milzzellen der Versuchstiere mit Myelomzellen fusioniert und entweder direkt in Kulturschalen oder als Ascites wachsen gelassen und sortiert.

Ob ein Antikörper gegen alpha-Amylase für die Erfindung geeignet ist, läßt sich leicht anhand einiger Vorversuche feststellen.

Das Screening-Verfahren besteht darin, lediglich festzustellen, ob der zu beurteilende monoklonale Antikörper überhaupt an das Enzym bindet, wenn bestimmte monoklonale Antikörper zugegen sind, welche diejenigen Bindungsstellen am Enzym besetzen, die keine im Sinne der Erfindung vorteilhafte Wirkung ergeben. Hierzu verwendet man die monoklonalen Antikörper, die von den Hybridzellklonen NCACC 84111301, NCACC 84122003, NCACC 85022203 und ECACC 86060601 gebildet werden. Eine mit diesen Antikörpern komplexierte alpha-Amylase bindet offenbar nur noch solche Antikörper, welche die erfindungsgemäße Wirkung ausüben.

Durch den erfindungsgemäßen Zusatz von monoklonalen Antikörpern gegen die alpha-Amylase wird überraschenderweise eine teilweise erhebliche Steigerung der Enzymaktivität erzielt und zwar für beide Isoenzyme der alpha-Amylase. Diese Steigerung eines Isoenzyms bleibt überraschenderweise auch dann erhalten, wenn das andere Isoenzym durch Zusatz spezifisch dieses Isoenzym hemmender monoklonaler Antikörper ausgeschaltet wird. In der nachstehenden Tabelle 1 wird der Einfluß verschiedener monoklonaler Antikörper aus Ascites auf die Aktivitäten der humanen Speichel-(h-SA) und Pankreas-alpha-Amylase (h-PA) bei verschiedenen Substraten gezeigt. Außerdem wird durch die erfindungsgemäße Arbeitsweise auch das Spaltmuster der Substrate vorteilhaft verändert derart, daß ein größerer Anteil an direkt bestimmbaren Spaltprodukten gebildet wird als ohne den Zusatz der monoklonalen Antikörper. Dies wird in der nachfolgenden Tabelle 2 gezeigt, in der für zwei verschiedene Substrate jeweils Aktivität und Zusammensetzung der Spaltprodukte ohne und mit erfindungsgemäß verwendeten monoklonalen Antikörper dargestellt wird.

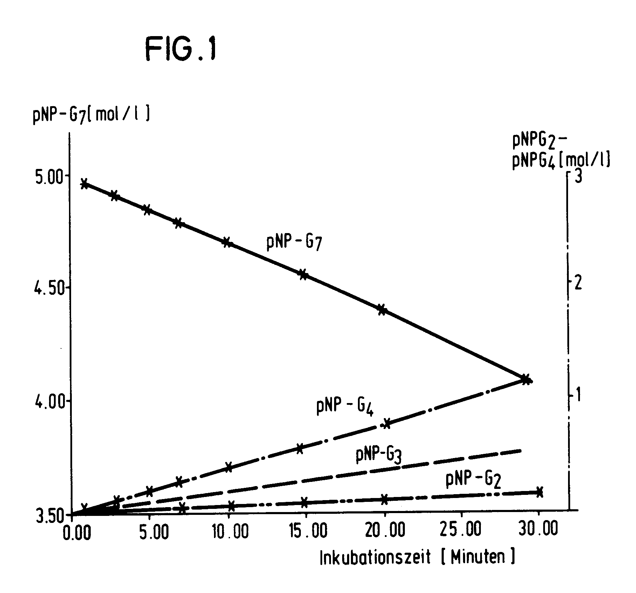

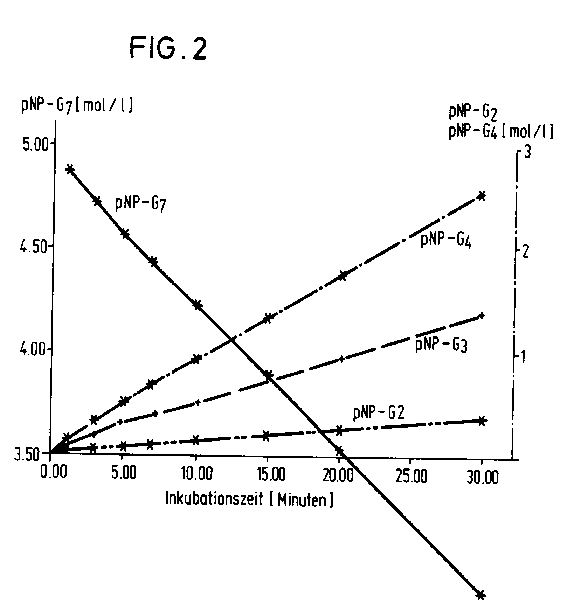

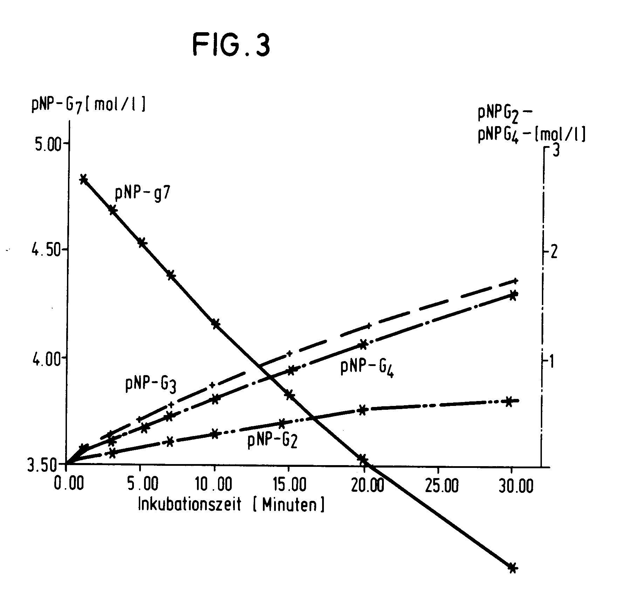

Die für die drei ersten Versuche gemäß Tabelle 2 ermittelten Spaltmuster sind in den Fig. 1 bis 3 der beigefügten Zeichnung graphisch dargestellt und lassen so deutlich den durch die Erfindung erzielten überraschenden Effekt erkennen.

Tabelle 1: Einfluß verschiedener monoklonaler Antikörper aus Ascites auf die Aktivitäten der humanen Speichel (h-SA) und Pankreas-alpha-Amylase (h-PA) in Kombination mit verschiedenen Substraten (vgl. Beispiel 1)

| MAKs der Hybridzell-klone | $G_5$-pNP[1] h-SA[5] | h-PA[6] | $G_6$-pNP[2] h-SA | h-PA | $G_7$-pNP[3] h-SA | h-PA | ethG$_7$-pNP[4] h-SA | h-PA |
|---|---|---|---|---|---|---|---|---|
| ohne | 100% | 100% | 100% | 100% | 100% | 100% | 100% | 100% |
| ECACC 86020602 | 115% | 116% | 108% | 119% | 121% | 122% | 131% | 128% |
| ECACC 86020601 | 118% | 154% | 142% | 164% | 175% | 188% | 148% | 156% |
| ECACC 86020602+ | – | 123% | 13% | 130% | 19% | 121% | 16% | 128% |
| ECACC 86020601+ | 4% | 158% | 4% | 171% | 4% | 178% | 3% | 148% |
| + | 4% | 99% | 2% | 88% | 3% | 97% | 3% | 100% |
| NACC 84122003 | 7% | 99% | 10% | 95% | 8% | 100% | 8% | 99% |

1) = p-Nitrophenyl-maltopentaose

2) = p-Nitrophenyl-maltohexaose

3) = p-Nitrophenyl-maltoheptaose

4) = p-Nitrophenyl-ethylidenmaltoheptaose

5) = human-Speichel-alpha-Amylase

6) = human-Pankreas-alpha-Amylase

+ = (+) NACC 84 122 003 + NACC 84 111 301

EP 0 251 195 B1

EP 0 251 195 B1

Tabelle 2: Einfluß der monoklonalen Antikörper ECACC Nr. 86020601 und 86020602 auf die humane Pankreas-alpha-Amylase, auf das Spaltmuster sowie die Reaktionsgeschwindigkeit der Substrate $G_7$-pNP und ethG$_7$-pNP (vgl. Beispiel 2)

| Substrat | MAK aus Hybridzell-klon ECACC Nr. | v, Abnahme der Substrate $\frac{\Delta mMol}{1 \cdot min}$ | % Akt., bezogen auf die Aktivität ohne MAK | Anteil verschiedenen Produkte | | | Fig.Nr. |
|---|---|---|---|---|---|---|---|
| | | | | $G_4$-pNP[3] | $G_3$-pNP[4] | $G_2$-pNP[5] | |
| $G_7$-pNP[1] | – | $3,0 \cdot 10^{-2}$ | 100 % | 63 % | 29 % | 8 % | 1 |
| $G_7$-pNP | 86020602 | $7,4 \cdot 10^{-2}$ | 243 % | 60 % | 31 % | 9 % | 2 |
| $G_7$-pNP | 86020601 | $7,5 \cdot 10^{-2}$ | 247 % | 41 % | 43 % | 16 % | 3 |
| ethG$_7$-pNP[2] | – | $1,4 \cdot 10^{-2}$ | 100 % | 22 % | 41 % | 37 % | |
| ethG$_7$-pNP | 86020602 | $3,6 \cdot 10^{-2}$ | 257 % | 19 % | 44 % | 37 % | |
| ethG$_7$-pNP | 86020601 | $4,3 \cdot 10^{-2}$ | 307 % | 10 % | 40 % | 50 % | |

1) = p-Nitrophenyl-maltoheptaose
2) = p-Nitrophenyl-ethylidenmaltoheptaose
3) = p-Nitrophenyl-maltotetraose
4) = p-Nitrophenyl-maltotriose
5) = p-Nitrophenyl-maltobiose

Ein weiterer Gegenstand der Erfindung ist ein Reagenz zur Bestimmung von alpha-Amylase, welches ein Substrat, ein oder mehrere Hilfsenzyme und ein System zur Messung eines Spaltproduktes enthält und durch einen Gehalt an mindestens einem monoklonalen Antikörper gegen alpha-Amylase gekennzeichnet ist, welcher in der Lage ist, alpha-Amylase in Gegenwart der monoklonalen Antikörper zu binden, die von den Hybridzellklonen NCACC 84111301, NCACC 84122003, NCACC 85022203 und ECACC 86060601 gebildet werden. Als monoklonalen Antikörper enthält es vorzugsweise den durch die Hybridzellklone 469A12 = ECACC 86020601 oder/und 472G12 } ECACC 86020602 gebildeten monoklonalen Antikörper.

Hinsichtlich der geeigneten Substrate und Hilfsenzyme gelten die oben zum Verfahren gemachten Ausführungen entsprechend. Falls das Reagenz für die Bestimmung eines der Isoenzyme der alpha-Amylase verwendet wird, enthält es zusätzlich einen oder mehrere monoklonale Antikörper gegen das jeweils andere alpha-Amylaseisoenzym. Bevorzugt wird dabei für ein Reagenz zur Bestimmung der Pankreas-alpha-Amylase ein Zusatz von monoklonalen Antikörpern gegen Speichel-alpha-Amylase, die durch den Hybridzellklon NCACC 84122003 gebildet werden und evtl. zusätzlich ein Zusatz von monoklonalen Antikörpern, die durch den Hybridzellklon NCACC 84111301 gebildet werden.

Durch die Erfindung wird erreicht, daß die Aktivität der alpha-Amylase je nach Substrat um 30 bis mehr als 100 % erhöht und gleichzeitig das Spaltmuster verbessert wird. Dadurch wird ein empfindlicherer Test sowohl auf Gesamt-alpha-Amylase als auch auf die Isoenzyme, insbesondere die Pankreasamylase geschaffen.

Die folgenden Beispiele erläutern die Erfindung weiter.

Beispiel 1:

I: Immunisierung von Mäusen mittels humaner Speichel-alpha-Amylase

Materialien

- Balb/c-Mäuse, weiblich, ca. 6-8 Wochen alt
- humane Speichel-alpha-Amylase (Sigma, Bestell Nr. A 0521)
- Freundsches Adjuvanz, komplett (Sigma, Nr. F 5881)
- Freundsches Adjuvanz, nicht komplett (Sigma, Bestell-Nr. F 5506)
- physiologische Kochsalzlösung (Sigma, Nr. P 8033)

Reagenzien

A: 300 $\mu$g/ml Speichel-alpha-Amylase in komplettem Freundschen Adjuvanz
B: 150 $\mu$g/ml Speichel-alpha-Amylase in nicht komplettem Freundschen Adjuvanz
C: 150 $\mu$g/ml Speichel-alpha-Amylase in physiologischer Kochsalzlösung

Durchführung

Balb/c Mäuse werden mit 300 $\mu$l A immunisiert. In ca. 8-wöchigem Rhythmus wird mit je 300 $\mu$l B 3 - 4 mal weiterimmunisiert. Vier Tage vor Fusion wird die letzte Immunisierung mit 300 $\mu$l C intravenös durchgeführt.

II: Fusion der Milzzellen

Die Fusion der Milzzellen mit Ag 8.653 (ATCC CRL 1580) oder SP2/0 (ATCC CRL 1581) Myelomzellen wird nach dem Standartverfahren gemäß J. of Immun. Meth. Vol. 39, 285-308 durchgeführt. Das Fusionsverhältnis Milz- zu Myelom-Zellen ist 5:1. Die Fusionsprodukte werden auf 20 24er-Kulturschalen (Costar) ausgesät und mit 5x10$^4$ peritoneal-exsudat-Zellen pro Kulturnapf gefüttert. Positive Primärkulturen (siehe C) werden 3 - 4 Wochen nach Fusion mit Hilfe eines Fluoreszenz-aktivierten Zellsorters kloniert. Die Zellen werden einzeln in 96er Costar-Platten abgelegt und mit 2x10$^4$ peritoneal-exsudat-Zellen gefüttert. Als Kulturmedium wird handelsübliches RPMI-1640-Medium mit 10 % fötalem Kälberserum (beschrieben in J.A.M.A. 199 (1957) 519) verwendet.

III: Screening-System für alpha-Amylase aktivierende Antikörper

Um die Konzentration amylaseaktivierender Antikörper im Serum immunisierter Mäuse oder im Kulturüberstand der Hybrid-Zellen oder in Ascites zu erfassen, wird ein Amylase-Aktivierungstext als Screening-Assay verwendet.

Dazu werden in 96er ELISA-Platten (Nunc, Dänemark) 50 $\mu$l Pankreas- oder Speichel-alpha-Amylase (etwa 400 U/l) in Puffer (50 mmol/l Tris-HCl, 1 % Rinderserumalbumin, 0,1 Mol/l NaCl, pH 7.5) vorgelegt und mit 50 $\mu$l der zu testenden Lösung (z.B. Kulturüberstand, Serum in verschiedener Verdünnung (mit physiologischer Kochsalzlösung), Ascites in verschiedener Verdünnung (mit physiologischer Kochsalzlösung)) 20 Minuten bei Raumtemperatur vorinkubiert.

Danach wird mit 50 $\mu$l Amylasesubstrat (4-Nitrophenylmaltoheptaosid mit alpha-Glucosidase; Boehringer Mannheim GmbH, Katalog-Bestell-Nr. 568589) die Enzymreaktion gestartet. Nach 20 - 60 Minuten bei Raumtemperatur werden die Extinktionen bei 405 nm in einem Photometer (ELISA-Reader, Kontron, Schweiz) bestimmt (Wert 1).

Folgende Kontrolle wird mitbestimmt: frisches Kulturmedium (Wert II). Die Aktivierung wird in % angegeben, bezogen auf Wert II:

$$\frac{\text{Wert I}}{\text{Wert II}} \cdot 100\ \% = \%\ \text{Aktivierung}$$

Folgende Ergebnisse werden mit dem Testsystem erzielt:

| Kulturüberstand des Klons | ECACC * | ECACC ** | NCACC 84122003 | NCACC 84111301 | Kontrolle Kulturmed |
|---|---|---|---|---|---|
| % Aktivierung der Speichel-amylase | 120 % | 175 % | 9 % | 98 % | 100 % |
| % Aktivierung der Pankreas-amylase | 122 % | 188 % | 102 % | 97 % | 100 % |

\* = 86020602        \*\* = 86020601

Aus ca. 2 % der Primärkulturen können aktivierende monoklonale Antikörper gefunden werden.

IV: Produktion von Ascites

1-2x10⁶ Hybridzellen (der Zell-Linie 86020601 oder 86020602) werden intraperitoneal in 1 bis 2 mal mit je 0,5 ml "Pristane Oil" (Sigma, München, Katalog-Bestell-Nr. T 7640) vorbehandelte Balb/c Mäuse (ca. 3 Monate alt) gespritzt. Nach ca. 15 - 20 Tagen werden pro Maus 3 bis 5 ml Ascites gezapft, der etwa 10 mg/ml IgG enthält.

Beispiel 2

Einfluß der aktivierenden MAKs ECACC 86020602 und ECACC 86020601 auf die Aktivität der humanen Speichel-alpha-Amylase und der humanen Pankreas-alpha-Amylase, auch in Kombination mit den MAKs NCACC 84122003 und NCACC 84111301.

Substanzen:

- EnzAmyl K-Test auf alpha-Amylase (Goedecke, Berlin; Kat. Best. Nr. 847 903); Substrat = Altotetraose
- Phadebas-Test auf alpha-Amylase (Pharmacia, Freiburg; Kat. Best. Nr. 51-5221-00/1); Substrat = blaugefärbte, hochmolekulare Stärke
- 4-Nitrophenyl-alpha-D-maltotetraosid, $G_4$-pNP (Boehringer Mannheim GmbH, Kat.Best. Nr. 720 518)
- 4-Nitrophenyl-alpha-D-maltopentaosid, G5-pNP (Boehringer Mannheim GmbH, Kat. Best. Nr. 720 496)
- 4-Nitrophenyl-alpha-D-maltohexaosid, $G_6$-pNP (Boehringer Mannheim GmbH, Kat. Best. Nr. 720 488)
- 4-Nitrophenyl-alpha-D-maltoheptaosid, $G_7$-pNP (Boehringer Mannheim GmbH, Kat. Best. Nr. 720 470)
- 4,6-Ethyliden-4-Nitrophenyl-alpha-D-maltoheptaosid, eth$G_7$-pNP (präpariert entsprechend Patentanmeldung DE-A 3328616)

- Ascites aus Mäusen der Zellinien ECACC 86020602, ECACC 86020601, NCACC 84122003, NCACC 84111301 (produziert analog Beispiel 1, IV).
- humane Speichel-alpha-Amylase (Sigma, München, Kat. Best. Nr. A 0521)
- humane Pankreas-alpha-Amylase (präpariert nach D.J. Stiefel und P.J. Keller (1973) Biochim. Biophys. Acta 302, 345 - 361)
- Rinderserumalbumin (RSA) (Boehringer Mannheim GmbH, Kat. Best.Nr. 238 031)
- alpha-Glucosidase (Boehringer Mannheim GmbH, Kat. Best. Nr. 750 590)

Reagenzien:

A: PBS: 100 mM Phosphatpuffer, 150 mM NaCl, pH = 7,1
B: PBS/RSA: PBS mit 6 % Rinderserumalbumin
C: h-SA-Lösung: humane Speichel-alpha-Amylase in PBS/RSA, 500 U/l (bestimmt bei 25°C mit handelsüblichem Reagenz alpha-Amylase PNP, Automatenpackung, Boehringer Mannheim GmbH, Kat. Best. Nr. 568 589)
D: h-PA-Lösung: humane Pankreas-alpha-Amylase in PBS/BSA, 500 U/l (bestimmt wie h-SA-Lösung)
E: Amylase-Reagenz (mit jeweils einem pNP-Substrat):

| | |
|---|---|
| 100 mMol/l | Hepes-Puffer; pH 7,1 |
| 50 mMol/l | NaCl |
| 5 mMol/l | pNP-Substrat (jeweils ein Substrat) |
| 30 U/l | alpha-Glucosidase |

F: Ascitesverdünnungen: Die verschiedenen Asciti werden jeweils 1 + 50 mit PBS/RSA verdünnt.

Durchführung:

1. Vorbereitung der Amylase mit MAKs aus Ascites
   - 100 µl Amylase (h-SA oder h-PA) werden mit 10 µl der verschiedenen Ascitesverdünnungen (einer oder mehrere) gemischt und auf 200 µl mit PBS/RSA aufgestockt. Als Kontrolle dient Amylase, die nur mit PBS/RSA aufgestockt wird.
   - Die Mischungen werden 5 Minuten bei Raumtemperatur inkubiert.
2. Bestimmung der Amylaseaktivität mit dem Substrat $G_4$
   - Die Bestimmung der Amylaseaktivität der Amylase-MAK-Mischungen bzw. der entsprechenden Kontrollen erfolgt mit dem Goedecke EnzAmyl K-Test bei 25°C genau nach Anleitung.
3. Bestimmung der Amylaseaktivität mit hochmolekularem Substrat
   - Die Bestimmung der Amylaseaktivität der Amylase-MAK-Mischungen bzw. der entsprechenden Kontrollen erfolgt mit dem Phadebas-Test der Firma Pharmacia genau nach Anleitung
4. Bestimmung der Amylaseaktivität mit den PNP-Substraten
   - 1 ml der Amylase-Reagenzien E werden in Halbmikroküvetten auf 25°C temperiert.
   - Je 50 µl der Amylase-MAK-Mischungen bzw. Kontrollen werden dazugegeben und gut gemischt.
   - Es erfolgt die Bestimmung der Extinktionsänderung $\Delta E/\min$ zwischen der 3. und 7. Minute nach Amylase-MAK-Zugabe bei 405 nm.

Berechnung

1. $G_4$-Substrat und hochmolekulares Substrat
   - Die als Einheiten pro Liter (U/l) erhaltenen Amylaseaktivitäten der Amylase-MAK-Mischungen werden auf die entsprechenden Amylaseaktivitäten der Kontrollen (ohne MAKs) bei gleichem Substrat bezogen und als %-Werte angegeben.
2. pNP-Substrate
   - Die als $\Delta E/\min$ erhaltenen Amylaseaktivitäten der Amylase-MAK-Mischungen werden auf die entsprechenden Amylaseaktivitäten der Kontrollen (ohne MAKs) bei gleichem pNP-Substrat bezogen und als %-Werte angegeben.

Ergebnisse

8

Die Einflüsse der monoklonalen Antikörper auf die Aktivitäten der Speichel- und Pankreasamylase sind in der Tabelle 1 bei den verschiedenen Substraten als %-Werte Aktivität bezogen auf die Aktivität ohne MAKs zusammengefaßt.

Beispiel 3

Bestimmung des Spaltmusters von $G_7$-pNP und ethG$_7$-pNP durch humane Pankreas-alpha-Amylase mit und ohne monoklonale Antikörper ECACC 86020601 und ECACC 86020602 sowie der Reaktionsgeschwindigkeit der Spaltung.

Substanzen

- 4,6-Ethyliden-4-Nitrophenyl-alpha-D-maltoheptaosid, ehtG$_7$-pNP (präpariert entsprechend Patentanmeldung DE-A 3328616)
- 4-Nitrophenyl-alpha-D-maltoheptaosid, $G_7$-pNP (Boehringer Mannheim GmbH, Kat. Best. Nr. 720 470)
- Ascites der Zellinie ECACC 86020601
- Ascites der Zellinie ECACC 86020602
- humane Pankreas-alpha-Amylase (präpariert nach D.J. Stiefel und P.J. Keller (1975) Biochim. Biophys. Acta 302, 345 - 361)
- Rinderserumalbumin (RSA)
  (Boehringer Mannheim GmbH, Kat. Best. Nr. 238 031)

Reagenzien

A: 150 mM Hepes-Puffer, 50 mM NaCl, 1 mg/ml NaN$_3$, pH 7,1
B: 100 mM Phosphatpuffer, 150 mM NaCl, 6 % RSA, pH 7,1
C: humane Pankreas-alpha-Amylase in Puffer B, 756 U/l (bestimmt bei 25°C mit handelsüblichem Reagenz-alpha-Amylase pNP, Automatenpackung, Boehringer Mannheim GmbH, Kat. Best. Nr. 568 589)
D: monoklonale Antikörper ECACC 86020601: Ascites 1 + 50 verdünnt mit B
E: monoklonale Antikörper ECACC 86020602: Ascites 1 + 50 verdünnt mit B
F: 20 Teile Acetonitril, gemischt mit 3 Teilen 1 Mol/l H$_3$PO$_4$
G: 1 Teil 25 mMol/l K$_2$HPO$_4$, pH = 7,1, gemischt mit 1 Teil Acetonitril
H: 5 mM ethG$_7$-pNP in A
I: 5 mM G$_7$-pNP in A

Durchführung

1. Mischung von Amylase und MAKs
   - 20 μl MAK-Lösung D oder E oder Puffer B werden mit 200 μl Pankreasamylase C gemischt und 5 Minuten bei Raumtemperatur inkubiert.
2. Reaktion von Amylase/MAK mit Substrat
   - 2 ml Substratlösung (H oder I) werden vorgelegt und auf 25 °C temperiert.
   - Die temperierte Substratlösung wird mit 200 μl der verschiedenen Amylase/MAK (Puffer) Mischungen (aus 1.) gestartet.
   - Nach genau 1, 3, 5, 7, 10, 20, 30 Minuten wurden jeweils 200 μl Aliquots der gestarteten Substratlösung entnommen und zu 130 μl Stoppreagenz F gegeben.
   - Die gestoppten Aliquots werden mit 1 ml der Lösung verdünnt und bei -20°C gelagert.
3. Bestimmung des Substrates und der Bruchstücke des Substrates durch HPLC
   - Die gestoppten, verdünnten Aliquots aus 2. werden aufgetaut.
   - Der Gehalt an G$_7$-pNP bzw. ethg$_7$-pNP, wobei an pNP und G$_1$-pNP bis G$_6$-pNP (der einzelnen Aliquots) wird durch HPLC genau wie in E.-O. Hägele et. al. (1982) Clin. Chem. 28/11, 2201 - 2205 beschrieben bestimmt.

Ergebnisse

Im Laufe der Reaktion der Amylase mit dem Substrat nimmt der Gehalt an Substrat ab. Tabelle 2 zeigt die mMol/(1 min) für die beiden Substrate ohne MAKs sowie mit dem MAKs ECACC 86020601 und ECACC 86020602. Weiterhin sind die Aktivitätswerte der Pankreasamylase mit MAKs normiert auf die Aktivitäten ohne MAKs dargestellt. Es entsteht bei den Reaktionen kein freies pNP, kein $G_1$-pNP, sowie kein $G_5$-pNP und $G_6$-pNP. Die %-Zahlen der anderen Bruchstücke sind ebenfalls in Tabelle 1 zusammengefaßt.

Abbildungen 1 bis 3 zeigen die Abnahmegeschwindigkeit des Substrats sowie die Zunahme der Produkte bei der Pankreasamylasereaktion für $G_7$-pNP als Substrat mit und ohne MAK.

Beispiel 4

Aktivierung von humaner alpha-Amylase durch die MAKs ECACC 86020601 und ECACC 86020602 beim Substrat Maltoheptaose

Substanzen:

- Monotest alpha-Amylase UV (Boehringer Mannheim GmbH, Mannheim, Katalog-Bestell-Nr. 240001)
- Ascited aus Mäusen der Zellinien ECACC 86020602 oder ECACC 86020601 (gemäß Beispiel 1)
- humane Speichel-alpha-Amylase (Sigma, München, Katalog-Bestell-Nr. A 0521)
- humane Pankreas-alpha-Amylase (präpariert nach D.J. Stiefel und P.J. Keller (1973) Biochem. Biophys. Acta 302, 345-361)
- Rinderserumalbumin, RSA (Boehringer Mannheim GmbH, Katalog-Bestell-Nr. 238031)

Reagenzien

A: PBS/RSA: 100 mMol/l Phosphatpuffer, 150 mMol/l NaCl, 6 % RSA, pH 7.1
B: h-SA-Lösung: humane Speichel-alpha-Amylase in PBS/RSA 500 U/l (bestimmt bei 25°C mit handelsüblichem Reagenz alpha-Amylase PNP, Automatenpackung, Boehringer Mannheim GmbH, Katalog-Bestell-Nr. 568589)
C: h-PA-Lösung: humane Pankreas-alpha-Amylase in PBS/RSA, 500 U/l (bestimmt wie h-SA-Lösung)
D: Ascitesverdünnung des MAK ECACC 86020601: Ascites 1 + 50 verdünnt mit PBS/RSA
E: Ascitesverdünnung des MAK ECACC 86020602: Ascites 1 + 50 verdünnt mit PBS/RSA

Durchführung

- alpha-Amylase und MAKs bzw. Puffer werden nach folgendem Schema gemischt:

| Lösung Probe | A | B | C | D | E |
|---|---|---|---|---|---|
| I | 10 µl | 100 µl | – | – | – |
| II | 10 µl | – | 100 µl | – | – |
| III | – | 100 µl | – | 10 µl | – |
| IV | – | 100 µl | – | – | 10 µl |
| V | – | – | 100 µl | 10 µl | – |
| VI | – | – | 100 µl | – | 10 µl |

Die Proben I - VI werden 10 Minuten bei Raumtemperatur inkubiert.
- Die Aktivität der Proben I - VI werden bei 25°C mit dem Monotest-alpha-Amylase UV genau nach Anleitung bestimmt.

Berechnung

Die bestimmten Aktivitäten der MAK-Amylase-Mischungen (Proben III - VI) werden auf die entsprechenden Aktivitäten der Amylase-Puffer-Mischungen (Probe I und Probe II) bezogen und als % Werte angegeben (Proben III und IV werden auf Probe I bezogen und Probe V und VI werden auf Probe II bezogen).

Ergebnis:

Die Einflüsse der monoklonalen Antikörper und des Puffers auf die humane Speichel- und Pankreas-alpha-Amylase ist in nachfolgender Tabelle dargestellt:

| Reagenz | % Aktivität der | |
|---|---|---|
| | humanen Speichel-alpha-Amylase | humanen Pankreas-alpha-Amylase |
| PBS/RSA | 100 % | 100 % |
| MAK ECACC 86020601 | 111 % | 125 % |
| MAK ECACC 86020602 | 113 % | 120 % |

Beispiel 5

Screening System auf monoklonale Antikörper aus Kulturüberstand von Fusionen oder aus Ascites oder aus Serum von Mäusen, die nicht die Epitope der humanen-alpha-Amylase Nummer I, II, III und V erkennen.

Nachfolgendes Beispiel zum Screening auf Amylase-aktivierende MAKs nutzt nicht die aktivierende Eigenschaft der MAKs zum Suchen aus. Es wird angenommen, daß die Amylase mindestens 5 Epitope hat (I-V). An all diese Epitope können MAKs gleichzeitig binden, Epitop IV stellt das Epitop dar, welches die aktivierenden und spaltmusterverändernden MAKs bindet.

Substanzen:

- Polyklonales Antiserum gegen Maus Fc-gamma-Fragmente aus Schafen
- Rinderserumalbumin (RSA) (Boehringer Mannheim GmbH, Mannheim Kat. Best. Nr. 238031)
- humanes Speichel-alpha-Amylase-Meerrettich-Peroxidase-Konjugat (h-SA = POD); präpariert nach M.B. Wilson und P.K. Nakam (in: Immunofluorescence and Related Staining Techniques, W. Knapp et al., Eds., Elsevier/-North-Holland Biomedical Press, Amsterdam-New York, 1978, pp 215-224) aus humaner Speichel-alpha-Amylase (Sigma, München, Kat. Best. Nr. A0521) und Meerrettich-Peroxidase (Boehringer Mannheim Gmbh, Mannheim, Kat. Best. 238031)
- Kulturüberstand des Hybridzellklons NCACC 84111301
- Fab'-Fragmente des monoklonalen Antikörpers produziert durch den Hybridzellklon NCACC 84111301 (MAK gegen Amylaseepitop I)

- Fab'-Fragmente des monoklonalen Antikörpers produziert durch den Hybridzellklon NCACC 84122003 (MAK gegen Amylaseepitop II)
- Fab'-Fragmente des monoklonalen Antiköpers produziert durch den Hybridzellklon NCACC 85022203 (MAK gegen Amylaseepitop III)
- Fab'-Fragmente des monoklonalen Antikörpers produziert durch den Hybridzellklon ECACC 86060601 (115C9) (MAK gegen Amylaseepitop V)
- ELISA-Platten (Nunc, Roskilde, Dänemark)
- ABTS®: 2,2-Azino-di-[3-ethylbenzthiazolinsulfonat(6)] (Boehringer Mannheim GmbH, Kat.Best. Nr. 102946)
- Chemikalien höchster Reinheit von Merck, Darmstadt oder Boehringer Mannheim GmbH
- Schutzfolie für ELISA Platten: Plate Sealers Cat. No. M30 (Dynatech Deutschland GmbH, Denkendorf)

Die Herstellung der Fab'-Fragmente der verschiedenen monoklonalen Antikörper erfolgte folgendermaßen: Zunächst wurde Ascites (analog zu Beispiel 1/IV) der verschiedenen Hybridzellklone produziert. Aus dem Ascites wurden über fraktionierte Ammoniumsulfatfällung und über eine Diethylaminoethylcellulose-Säule (nach A. Johnstone und R. Thorpe; in: Immunochemistry in Practice, Blackwell Scientific Publications, Oxford-London-Edinburgh-Boston-Melbourne, 1982, pp 43-47) die monoklonalen Antikörper aufgereinigt. Die Spaltung der Antikörper zu ihren Fab'-Fragmenten erfolgte nach A. Nisonoff (Methods Med. Res. 10, 134 ff (1964)).

Reagenzien:

A: Natriumcarbonatpuffer, 50 mM, pH 9,3 mit 10 μg/ml polyklonalen Antikörpern von Schafen gegen Maus Fc-gamma-Fragmente (gereinigt aus polyklonalem Antiserum über Ammoniumsulfatfällung und Diethylaminoethylcellulosechromatographie (A. Johnstone und R. Thorpe; in: Immunochemistry in Practice, Blackwell Scientific Publications, Oxford-London-Edinburgh-Boston-Melbourne, 1982, pp 43-47))
B: PBS/RSA: 100 mM Phosphatpuffer, 150 mM NaCl, pH = 7,1 mit 1 % Rinderserumalbumin (RSA)
C: Reagenz B mit 200 IU/l (Peroxidase-Einheiten) h-SA = POD und je 100 μg/ml der 4 Fab'-Fragmente (der Hybridzellklone NCACC 84111301, 84122003, 85022203 und ECACC 86060601 (76C8)); die Lösung wird 1 h vor Gebrauch angesetzt.
D: Peroxidase-Substratlösung: 100 mM Phosphat-Citrat-Puffer, pH = 4,4 mit 3,2 mM Natriumperborat und 1,9 mM ABTS®.
E: PBS: 100 mM Phosphatpuffer, 150 mM NaCl, pH = 7,1

Durchführung:

1. In jedes Tüpfel einer ELISA-Platte werden je 100 μl des Reagenzes A pipettiert. Die Platten werden mit einer Schutzfolie verschlossen und 18 h bei 4°C inkubiert.
2. Jeder Tüpfel der Platten wird 3x mit mindestens 250 μl Reagenz E gewaschen (Raumtemperatur, jeweils ca. 5 Minuten).
3. Jeder Tüpfel der Platten wird mit 250 μl Reagenz B inkubiert (Schutzfolie; Raumtemperatur, 1h).
4. Jeder Tüpfel der Platten wird wie unter 2. beschrieben gewaschen.
5. Kulturüberstand der verschiedenen Hybridzellklone oder Ascites oder Seren von immunisierten Mäusen werden unverdünnt und/oder in verschiedenen Verdünnungen (mit Reagenz B verdünnt) in verschiedene Tüpfel der Platten pipettiert (jeweils 100 μl). Die Platten werden verschlossen und 2 h bei 37°C inkubiert. Als Leerwert dienen Tüpfel, die nur mit Reagenz B inkubiert werden. Als Negativkontrolle wird Kulturüberstand des Hybridzellklons NCACC 83111301 verwendet.
6. Jeder Tüpfel der Platten wird wie unter 2. beschrieben gewaschen.
7. Jeder Tüpfel der Platten wird mit 100 μl Reagenz C (Amylase-POD-Konjugat + Fab'-Fragmente) inkubiert (Schutzfolie; 37°C, 2h).
8. Jeder Tüpfel der Platten wird 6x mit Reagenz E gewaschen (mindestens 250 μl, Raumtmperatur, jeweils ca. 2 Minuten). Die Tüpfel werden mittels einer Wasserstrahlpumpe und einer feinen Glaskanüle trockengesaugt.
9. Direkt danach wird in jedes Tüpfel des Platte 100 μl des Reagenzes D gegeben. Die Platten werden mit einer Schutzfolie verschlossen und bei 37 °C inkubiert (ca. 15-60 Minuten, je nach Konzentration der zu suchenden MAKs).

Auswertung:

Die Extinktionen der einzelnen Tüpfel der ELISA-Platten werden nach Entfernung der Schutzfolie in einem ELISA-Reader (Kontron, Schweiz) vermessen. Die Extinktionen der Leerwerte (nur Reagenz B) werden von den Extinktionen der anderen Tüpfel mit MAKs subtrahiert.

Tüpfel, die mit Antikörpern gegen das Epitop IV der Amylase beschichtet wurden, zeigen deutlich höhere Extinktionen als Tüpfel, die mit dem Kulturüberstand des Hybridzellklons NCACC 84111301 beschichtet wurden. Die Extinktionsunterschiede sollten mindestens 200 mE betragen.

Auf diese Art und Weise werden mit hoher Wahrscheinlichkeit alle MAKs gefunden, die das Epitop IV der Amylase erkennen. Diese MAKs haben überraschenderweise die Eigenschaft, die Amylase zu aktivieren (d.h. die Spaltgeschwindigkeit zu beschleunigen) und evtl. auch das Spaltmuster des Substrates zu verändern.

**Patentansprüche**

1. Verfahren zur Bestimmung von alpha-Amylase durch Spaltung eines Substrats in Gegenwart eines oder mehrerer Hilfsenzyme und Messung eines Spaltproduktes,
   **dadurch gekennzeichnet,**
   daß man die Umsetzung in Gegenwart von monoklonalen Antikörpern gegen alpha-Amylase durchführt und hierbei mindestens einen monoklonalen Antikörper verwendet, welcher in der Lage ist, alpha-Amylase in Gegenwart der monoklonalen Antikörper zu binden, die von den Hybridzellklonen NCACC 84111301, NCACC 84122003, NCACC 85022203 und ECACC 86060601 gebildet werden.

2. Verfahren nach Anspruch 1,
   **dadurch gekennzeichnet,**
   daß man als Substrat ein D-Maltooligosid mit 2 bis 10 Glucoseeinheiten, welches gegebenenfalls durch eine chromophore Gruppe substituiert ist oder/und mindestens eine Ethylidenschutzgruppe trägt, verwendet.

3. Verfahren nach Anspruch 2,
   **dadurch gekennzeichnet,**
   daß man ein D-Maltooligosid mit 4 bis 8 Glucoseeinheiten verwendet.

4. Verfahren nach einem der Ansprüche 2 oder 3,
   **dadurch gekennzeichnet,**
   daß ein D-Maltooligosid verwendet wird, welches als chromophore Gruppe einen Nitrophenylrest trägt.

5. Verfahren nach einem der Ansprüche 2 bis 4,
   **dadurch gekennzeichnet,**
   daß man als Hilfsenzym alpha-Glucosidase zusetzt.

6. Verfahren nach Anspruch 5,
   **dadurch gekennzeichnet,**
   daß man Maltoheptaose als Substrat und als zusätzliche Hilfsenzyme neben alpha-Glucosidase noch Hexokinase und Glucose-6-phosphatdehydrogenase verwendet.

7. Verfahren nach Anspruch 4 und 5,
   **dadurch gekennzeichnet,**
   daß man als weiteres Hilfsenzym neben alpha-Glucosidase noch $\beta$-Glucosidase verwendet.

8. Verfahren nach Anspruch 3,
   **dadurch gekennzeichnet,**
   daß man als Substrat Maltotetraose und als Hilfsenzyme Maltosephosphorylase, $\beta$-Phosphoglucomutase und Glucose-6-phosphatdehydrogenase verwendet.

9. Verfahren nach einem der vorhergehenden Ansprüche,
   **dadurch gekennzeichnet,**
   daß man einen monoklonalen Antikörper verwendet, der vom Hybridzellklon ECACC 86020601 oder ECACC 86020602 gebildet wird.

**10.** Verfahren nach einem der vorhergehenden Ansprüche,
**dadurch gekennzeichnet,**
daß man zur Bestimmung eines der beiden alpha-Amylaseisoenzmye zusätzlich einen monoklonalen Antikörper verwendet, der das jeweils andere alpha-Amylaseisoenzym spezifisch hemmt.

**11.** Reagenz zur Bestimmung von alpha-Amylase, enthaltend ein Substrat, ein oder mehrere Hilfsenzyme und ein System zur Messung eines Spaltproduktes,
**gekennzeichnet durch,**
einen Gehalt an mindestens einem monoklonalen Antikörper gegen alpha-Amylase und hierbei einen monoklonalen Antikörper verwendet, welcher in der Lage ist, alpha-Amylse in Gegenwart der monoklonalen Antikörper zu binden, die von den Hybridzellklonen NCACC 84111301, NCACC 84122003, NCACC 85022203 und ECACC 86060601 gebildet werden.

**12.** Reagenz nach Anspruch 11,
**dadurch gekennzeichnet,**
daß es den durch die Hybridzellklone ECACC 86020601 oder/und ECACC 86020602 gebildeten monoklonalen Antikörper enthält.

**13.** Reagenz nach Anspruch 11 oder 12,
**dadurch gekennzeichnet,**
daß es als Substrat ein D-Maltooligosid mit 2 bis 10 Glucoseeinheiten, welches gegebenenfalls durch eine chromophore Gruppe substituiert ist oder/und mindestens eine Ethylidenschutzgruppe trägt, enthält.

**14.** Reagenz nach Anspruch 13,
**dadurch gekennzeichnet,**
daß das Substrat 4 bis 8 Glucoseeinheiten enthält.

**15.** Reagenz nach Anspruch 13 oder 14,
**dadurch gekennzeichnet,**
daß das Maltooligosid als chromophore Gruppe einen Nitrophenylrest trägt.

**16.** Reagenz nach einem der Ansprüche 13 bis 15,
**dadurch gekennzeichnet,**
daß es als Hilfsenzym alpha-Glucosidase enthält.

**17.** Reagenz nach Anspruch 16,
**dadurch gekennzeichnet,**
daß es Maltoheptaose als Substrat und als Hilfsenzyme alpha-Glucosidase, Hexokinase und Glucose-6-phosphatdehydrogenase enthält.

**18.** Reagenz nach Anspruch 15 oder 16,
**dadurch gekennzeichnet,**
daß es alpha-Glucosidase und $\beta$-Glucosidase enthält.

**19.** Reagenz nach Anspruch 13,
**dadurch gekennzeichnet,**
daß es Maltotetraose als Substrat und Maltosephosphorylase, $\beta$-Phosphoglucomutase und Glucose-6-phosphatdehydrogenase als Hilfsenzyme verwendet.

**Claims**

**1.** Process for the determination of alpha-amylase by cleavage of a substrate in the presence of one or more auxiliary enzymes and measurement of a cleavage product, characterised in that one carries out the reaction in the presence of monoclonal antibodies against alpha-amylase and hereby uses at least one monoclonal antibody which is able to bind alpha-amylase in the presence of monoclonal antibodies which are formed by the hybrid cell clones NCACC 84111301, NCACC 84122003, NCACC 85022203 and ECACC 86060601.

2. Process according to claim 1, characterised in that, as substrate, one uses a D-maltooligoside with 2 to 10 glucose units, which is possibly substituted by a chromophoric group and/or carries at least one ethylidene protective group.

3. Process according to claim 2, characterised in that one uses a D-maltooligoside with 4 to 8 glucose units.

4. Process according to one of claims 2 or 3, characterised in that a D-maltooligoside is used which carries a nitrophenyl radical as chromophoric group.

5. Process according to one of claims 2 to 4, characterised in that one uses $\alpha$-glucosidase as auxiliary enzyme.

6. Process according to claim 5, characterised in that one uses maltoheptaose as substrate and, as additional auxiliary enzymes, besides alpha-glucosidase, also hexokinase and glucose-6-phosphate dehydrogenase.

7. Process according to claim 4 and 5, characterised in that, as further auziliary enzyme, besides $\alpha$-glucosidase, one also uses $\beta$-glucosidase.

8. Process according to claim 3, characterised in that, as substrate, one uses maltotetraose and, as auxiliary enzyme, maltose phosphorylase, $\beta$-phosphoglucomutase and glucose-6-phosphate dehydrogenase,

9. Process according to one of the preceding claims, characterised in that one uses a monoclonal antibody which is formed by the hybrid clone ECACC 86020601 or ECACC 86020602.

10. Process according to one of the preceding claims, characterised in that for the determination of one of the two alpha-amylase isoenzymes, one additionally uses a monoclonal antibody which specifically inhibits the other alpha-amylase isoenzyme.

11. Reagent for the determination of alpha-amylase containing a substrate, one or more auxiliary enzymes and a system for the measurement of a cleavage product, characterised by a content of at least one monoclonal antibody against alpha-amylase and hereby uses a monoclonal antibody which is able to bind alpha-amylase in the presence of the monoclonal antibodies which is formed by the hybrid clones NCACC 84111301, NCACC 84122003, NCACC 85022203 and ECACC 86060601.

12. Reagent according to claim 12, characterised in that it contains the monoclonal antibodies formed by the hybrid cell clones ECACC 86020601 and/or ECACC 86020602.

13. Reagent according to claim 11 or 12, characterised in that, as substrate, it contains a D-maltooligoside with 2 to 10 glucose units which is possibly substituted by a chromophoric group and/or carries at least one ethylidene protective group.

14. Reagent according to claim 13, characterised in that the substrate contains 4 to 8 glucose units.

15. Reagent according to claim 13 or 14, characterised in that the maltooligoside carries a nitrophenyl radical as chromophoric group.

16. Reagent according to one of claims 13 to 15, characterised in that it contains alpha-glucosidase as auxiliary enzyme.

17. Reagent according to claim 16, characterised in that it contains maltoheptaose as substrate and alpha-glucosidase, hexokinase and glucose-6-phosphate dehydrogenase as auxiliary enzymes.

18. Reagent according to claim 15 or 16, characterised in that it contains alpha-glucosidase and $\beta$-glucosidase.

15

EP 0 251 195 B1

19. Reagent according to claim 13, characterised in that it uses maltotetraose as substrate and maltose phosphorylase, $\beta$-phosphoglucomutase and glucose-6-phosphate dehydrogenase as auxiliary enzymes.

**Revendications**

1. Procédé de détermination de l'alpha-amylase par clivage d'un substrat en présence d'une ou plusieurs enzymes auxiliaires et mesure d'un produit de clivage, caractérisé en ce que l'on conduit la réaction en présence d'anticorps monoclonaux contre l'alpha-amylase et on utilise à cet effet au moins un anticorps monoclonal qui est capable de se lier à l'alpha-amylase en présence des anticorps monoclonaux qui sont formés par les clones de cellules hybrides NCACC 84111301, NCACC 84122003, NCACC 85022203 et ECACC 86060601.

2. Procédé selon la revendication 1, caractérisé en ce que l'on utilise somme substrat un D-malto-oligoside contenant 2 à 10 unités de glucose qui est éventuellement substitué par un groupe chromophore et/ou qui porte au moins un groupe protecteur d'éthylidène.

3. Procédé selon la revendication 2, caractérisé en ce que l'on utilise un D-malto-oligoside contenant 4 à 8 unités de glucose.

4. Procédé selon l'une des revendications 2 et 3, caractérisé en ce que l'on utilise un D-malto-oligoside qui porte un reste nitrophényle comme groupe chromophore.

5. Procédé selon l'une des revendications 2 à 4, caractérisé en ce que l'on utilise l'alpha-glucosidase comme enzyme auxiliaire.

6. Procédé selon la revendication 5, caractérisé en ce que l'on utilise le maltoheptaose comme substrat et comme enzymes auxiliaires supplémentaires l'hexokinase et la glucose-6-phosphate déshydrogénase en plus de l'alpha-glucosidase.

7. Procédé selon les revendications 4 et 5, caractérisé en ce que l'on utilise la $\beta$-glucosidase comme enzyme auxiliaire supplémentaire en plus de l'alpha-glucosidase.

8. Procédé selon la revendication 3, caractérisé en ce que l'on utilise comme substrat le maltotétraose et comme enzymes auxiliaires la maltose phosphorylase, la $\beta$-phosphoglucomutase et la glucose-6-phosphate déshydrogénase.

9. Procédé selon l'une des revendications précédentes, caractérisé en ce que l'on utilise un anticorps monoclonal qui est formé par le clone de cellules hybrides ECACC 86020601 ou ECACC 86020602.

10. Procédé selon l'une des revendications précédentes, caractérisé en ce que l'on utilise en outre pour la détermination de l'une des deux isoenzymes de l'alpha-amylase un anticorps monoclonal qui inhibe de manière spécifique l'autre isoenzyme de l'alpha-amylase.

11. Réactif pour la détermination de l'alpha-amylase, contenant un substrat, une ou plusieurs enzymes auxiliaires et un système pour mesurer un produit de clivage, caractérisé en ce qu'il contient au moins un anticorps monoclonal contre l'alpha-amylase et utilise à cet effet un anticorps monoclonal qui est capable de se lier à l'alpha-amylase en présence des anticorps monoclonaux qui sont formés par les clones de cellules hybrides NCACC 84111301, NCACC 84122003, NCACC 85022203 et ECACC 86060601.

12. Réactif selon la revendication 11, caractérisé en ce qu'il contient l'anticorps monoclonal formé par les clones de cellules hybrides ECACC 86020601 et/ou ECACC 86020602.

13. Réactif selon la revendication 11 ou 12, caractérisé en ce qu'il contient comme substrat un D-malto-oligoside contenant 2 à 10 unités de glucose, qui est éventuellement substitué par un groupe chromophore et/ou qui porte au moins un groupe protecteur d'éthylidène.

14. Réactif selon la revendication 13, caractérisé en ce que le substrat contient 4 à 8 unités de glucose.

16

**15.** Réactif selon la revendication 13 ou 14, caractérisé en ce que le malto-oligoside porte un reste nitrophényle comme groupe chromophore.

**16.** Réactif selon l'une des revendications 13 à 15, caractérisé en ce qu'il contient l'alpha-glucosidase comme enzyme auxiliaire.

**17.** Réactif selon la revendication 16, caractérisé en ce qu'il contient du maltoheptaose comme substrat et comme enzymes auxiliaires l'alpha-glucosidase, l'hexokinase et la glucose-6-phosphate déshydrogénase.

**18.** Réactif selon la revendication 15 ou 16, caractérisé en ce qu'il contient l'alpha-glucosidase et la $\beta$-glucosidase.

**19.** Réactif selon la revendication 13, caractérisé en ce qu'il utilise le maltotétraose comme substrat et la maltose phosphorylase, la $\beta$-phosphoglucomutase et la glucose-6-phosphate déshydrogénase comme enzymes auxiliaires.

FIG.1

# FIG.2

# FIG.3